(19) Europäisches Patentamt
European Patent Office
Office européen des brevets

(11) **EP 2 650 021 B1**

(12) **EUROPEAN PATENT SPECIFICATION**

(45) Date of publication and mention of the grant of the patent:
**09.12.2015 Bulletin 2015/50**

(21) Application number: **12760366.0**

(22) Date of filing: **10.02.2012**

(51) Int Cl.:
*A61K 47/34* (2006.01)     *A61F 2/16* (2006.01)
*A61K 9/08* (2006.01)     *A61K 31/496* (2006.01)
*A61K 31/5383* (2006.01)     *A61P 27/02* (2006.01)
*G02C 7/04* (2006.01)     *A61K 9/00* (2006.01)
*G02B 1/04* (2006.01)     *A61F 9/00* (2006.01)
*A61K 31/41* (2006.01)

(86) International application number:
**PCT/JP2012/053054**

(87) International publication number:
**WO 2012/127927 (27.09.2012 Gazette 2012/39)**

(54) **MEDICAL DEVICE FOR CONTROLLED RELEASE OF DRUG**

MEDIZINISCHE VORRICHTUNG ZUR GESTEUERTEN ABGABE EINER ARZNEI

DISPOSITIF MÉDICAL POUR LA LIBÉRATION CONTRÔLÉE DE MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.03.2011 JP 2011061735**

(43) Date of publication of application:
**16.10.2013 Bulletin 2013/42**

(73) Proprietor: **Seed Co., Ltd.**
**Bunkyo-ku, Tokyo 113-8402 (JP)**

(72) Inventors:
• **YAMAZAKI Keiko**
**Tokyo 113-8402 (JP)**
• **SYOUJI Kiyoshi**
**Tokyo 113-8402 (JP)**
• **MATSUNAGA Toru**
**Tokyo 113-8402 (JP)**
• **SATO Takao**
**Tokyo 113-8402 (JP)**

(74) Representative: **Gervasi, Gemma et al**
**Notarbartolo & Gervasi S.p.A.**
**Corso di Porta Vittoria 9**
**20122 Milano (IT)**

(56) References cited:
**EP-A2- 0 351 364     EP-A2- 1 128 191**
**DE-A1- 2 636 559     JP-A- 52 054 014**
**JP-A- 62 103 028     JP-A- H01 503 072**
**JP-A- 2004 107 253     JP-A- 2005 218 780**

• **SEAN BRAHIM ET AL.: 'Release Characteristics of Novel pH-Sensitive p(HEMA-DMAEMA) Hydrogels Containing 3-(Trimethoxy-silyl) Propyl Methacrylate' BIOMACROMOLECULES vol. 4, 2003, pages 1224 - 1231**
• **J C TILLER ET AL.: 'Reloadable antimicrobial coatings based on amphiphilic silicone networks' SURFACE COATINGS INTERNATIONAL PART B: COATINGS TRANSACTIONS vol. 88, no. BL, 2005, pages 49 - 53**

**Description**

Cross-Reference to Related Applications

The present application claims priority from Japanese Patent Application No. 2011-061735 filed on March 18, 2011.

Technical Field

The present invention relates to drug sustained-release medical devices. In particular, the present invention relates to a drug sustained-release medical device suitable for ophthalmic lenses.

Background Art

In drug treatment of various eye diseases, eye drops, suspension, eye ointment, and the like are used. The eye drops can be easily administered. However, medicinal substances in the eye drops are diluted by lacrimal fluid, and are promptly discharged through lachrymal ducts. In order to maintain the effective concentration of the drug, there is the problem that there is a need to increase a dose of medicinal substances in the eye drops, a need to increase the frequency of the administration of the eye drops or another need. In any case, patients need to bear a large burden.

In the case of the suspension and the eye ointment, medicinal substances can remain in eyes for a longer period of time compared with the eye drops. However, the administration of the suspension and the eye ointment entails side effects, such as to arouse eye irritation and to cloud field of view.

Moreover, the eye drops, the suspension, and the eye ointment all are likely to have adverse effects on contact lenses, such as distortion or alteration. The use of such formulations in treatment is not suitable for people wearing contact lenses.

In general, contact lenses are categorized into non-water content contact lenses and water content contact lenses. For the non-water content contact lenses, with the aim of improving oxygen permeability, silicone-based materials are generally employed as a component of lens materials. In recent years, even for the water content contact lenses, silicone-based materials that are higher in oxygen permeability have been employed as the component to ensure safety for eyes. Such water content contact lenses, which include silicone-based materials among the components thereof, are marketed as so-called silicone hydrogel contact lens (referred to as SHGCL, hereinafter). SHGCL is high in oxygen permeability, and does not inhibit the supply of oxygen to the cornea of a person wearing the contact lenses. SHGCL is also able to reduce eye damage caused by lack of oxygen. Ophthalmic lenses that take advantage of the above benefits are disclosed (See Japanese Unexamined Patent Application Publication (KOKAI) No. 2009-204770 as Patent Document 1): the ophthalmic lenses contain pranlukast as a medicinal substance, as well as silicone-containing monomer as a component thereof.

As for contact lenses that contain a medicinal substance and release the medicinal substance in a sustained manner after a person wears the contact lenses, for example, the drug sustained-release ophthalmic lenses are known (See Japanese Unexamined Patent Application Publication (KOKAI) No. 2004-305313 as Patent Document 2) : the drug sustained-release ophthalmic lenses, which are coordinated in the form of hydrogel, include a cationic medicinal substance, as well as methacrylate containing hydrophilic monomers and phosphate groups, and monomers containing nitrogen atom among the components thereof.

Prior Art Documents

Patent Documents

Patent Document 1: Japanese Unexamined Patent Application Publication (KOKAI) No. 2009-204770
Patent Document 2: Japanese Unexamined Patent Application Publication (KOKAI) No. 2004-305313
EP1128191A2 discloses contact lens comprising a copolymer of silicone-containing polymer, a water-insoluble monoolefin and a water soluble monoolefin e.g. methacrylate.
EP0351364A2 discloses amphiphilic hydrogel comprising 4 components, one of which comprises silicon-containing methacrylate.

Disclosure of the Invention

Problems to Be Solved by the Invention

Patent Document 1 discloses that the ophthalmic lenses described in Patent Document 1 can achieve medicinal effects for a relatively long period of time for allergic eye disease. However, Patent Document 1 does not disclose how to control the amount of medicinal substances released in a sustained manner and the sustained-release speed for the ophthalmic lenses described in Patent Document 1. In particular, preferable effects on the control of the drug sustained-release speed, which a silicone compound that is among the components of SHGCL shows, are not mentioned. Moreover, in view of the fact that the silicone compound is hydrophobic, it can be estimated that the amount of medicinal substances taken into the ophthalmic lenses described in Patent Document 1 is small, and that the ophthalmic lenses have limited medicinal effects in treatment.

The drug sustained-release ophthalmic lenses described in Patent Document 2 include hydrophilic monomers and polar monomers as components of hydrogel, thereby making it possible to release water-soluble medicinal substances inside the lenses in a sustained manner after a person wears the contact lenses. The reason is that the water-soluble medicinal substances are kept in the hydrogel due to ionic interactions in the hydrogel between ionic functional groups and water-soluble medicinal substances; the water-soluble medicinal substances are eluted into a water portion inside the hydrogel due to ion-exchange reaction at a time when a person wears the contact lenses; the water-soluble medicinal substances then flow into the lacrimal fluid due to exchange reaction (pumping action) between the water portion inside the hydrogel and the lacrimal fluid followed by reaching ocular tissues.

However, the ionic functional groups in the hydrogel can easily react to changes in the external environment, such as temperatures and pH. Thus, the hydrogel cannot keep the medicinal substances in a stable manner. That is, the problem with the ophthalmic lenses described in Patent Document 2 is that the contact lenses' performance of retaining medicinal substances at the time when the contact lenses is worn is lower than that at the time when the contact lenses is kept at ambient temperatures, and an initial burst, which is a phenomenon that the medicinal substances are released in high concentration immediately after a person begins to wear the contact lenses, is likely to occur. Moreover, poor oxygen permeability makes it difficult for a person to wear the contact lenses for a long period of time.

Therefore, a problem to be solved by the present invention, set up by the present inventors, is to provide a drug sustained-release medical device suitable for ophthalmic lenses, which is designed to reduce the amount of retained medicinal substances that are initially released compared with conventional drug sustained-release contact lenses, thereby being able to release a therapeutically effective dose of medicinal substances for a long period of time in a sustained manner, as well as to have the same level of oxygen permeability as conventional SHGCL.

Means of Solving the Problems

In order to solve the above problem, the present inventors considered that it is important to control the amount of medicinal substances flowing out into lacrimal fluid due to pumping action after the medicinal substances are released by ion-exchange reaction into a water portion inside the hydrogel. Furthermore, the present inventors came to the realization that, if the exchange efficiency between the water portion inside the hydrogel and the lacrimal fluid is lowered, the amount of medicinal substances flowing out may be controlled in an effective manner.

As a result of extensive studies, the present inventors conceived that, by letting an amphiphilic hydrogel, which includes a hydrophilic unit containing an ionic group and a hydrophobic unit that is a silicone compound, hold water-soluble medicinal substances, the release of the water-soluble medicinal substances held by ionic bond with the ionic group may be controlled by repulsion action against the hydrophobic unit. The present inventors also made a hypothesis that the sustained-release speed of the water-soluble medicinal substances may be controlled by controlling the composition of the hydrophilic and hydrophobic units.

However, when an ionic monomer is employed as the hydrophilic unit, it is difficult to make the ionic monomer compatible with the silicone-containing monomer that is employed as the hydrophobic unit because the ionic monomer is quite high in hydrophilicity. The present inventors further conducted research and development, and found a combination of the ionic monomer and silicone-containing monomer that are high in compatibility, and succeeded in obtaining a uniform monomer solution by using the combination. With the use of an amphiphilic hydrogel that is obtained by copolymerizing the monomer solution, the present inventors succeeded in creating a drug sustained-release medical device suitable for ophthalmic lenses, which is designed to reduce the amount of retained medicinal substances that are initially released compared with conventional drug sustained-release contact lenses, thereby being able to release a therapeutically effective dose of medicinal substances for a long period of time in a sustained manner, as well as to have the same level of oxygen permeability as conventional SHGCL. The present invention is one completed based on the findings described above.

Thus, according to the present invention, what is provided is a drug sustained-release medical device containing an amphiphilic hydrogel for retaining a medicinal substance, wherein a sustained release rate of the medicinal substance is less than or equal to 50 wt% 24 hours after a release of the medicinal substance begin according to claim 1.

When in the present invention "drug sustained-release medical device" is stated, it is meant "drug sustained-release medical contact lens" as in claim 1.

Preferably, the sustained release rate of the medicinal substance is less than or equal to 25 wt% four hours after the release of the medicinal substance begins.

Preferably, the amphiphilic hydrogel contains a copolymer of silicone-containing monomer and ionic monomer.

Preferably, the silicone-containing monomer contains one, two, or three monoalkylsiloxy groups, dialkylsiloxy groups, or trialkylsiloxy groups.

Preferably, the ionic monomer contains an anionic monomer.

According to another aspect of the present invention, what is provided is a drug sustained-release method of releasing in a sustained manner a medicinal substance that uses an amphiphilic hydrogel retaining the medicinal substance, by which a sustained release rate of the medicinal substance is less than or equal to 25 wt% four hours after a release of the medicinal substance begins, and is less than or equal to 50 wt% 24 hours after the release of the medicinal substance begins.

Effects of the Invention

Since the drug sustained-release medical device of the present invention is made of an amphiphilic hydrogel, which is for example an amphiphilic hydrogel containing a hydrophilic unit containing an ionic group and a hydrophobic unit that is a silicone compound, the sustained release speed of a water-soluble medicinal substance retained by ionic bond with the ionic group can be controlled by repulsion action against the hydrophobic unit.

In the drug sustained-release medical device of the present invention, the hydrophobic unit has a specific configuration. Therefore, while keeping the same level of oxygen permeability as conventional SHGCL, it is possible to release a therapeutically effective dose of medicinal substances for a long period of time in a sustained manner. Accordingly, for example, if the drug sustained-release medical device of the present invention is employed as an ophthalmic device, a patient affected by eye disease can wear the ophthalmic device for a long period of time, e.g. for 72 or more hours in a row, to cure the disease.

In the case of an ophthalmic device that is made of a silicone-containing monomer as described in Patent Document 1, the expansibility of water decreases, making it difficult to take in water-soluble medicinal substances. To the contrary, the ophthalmic device that is based on the drug sustained-release medical device of the present invention contains the ionic monomer that is highly compatible with the silicone-containing monomer, among the components thereof. Therefore, the ophthalmic device is high in hydrous properties (water absorbability), and can easily capture medicinal substances. Another advantage is that the ionic bond with the ionic monomer makes it difficult for the medicinal substances captured to be released in a sustained manner. Moreover, the amount of medicinal substances captured can be adjusted based on the amount of the ionic monomer blended.

Brief Description of the Drawings

FIG. 1 is a diagram showing a drug sustained-release rate at a measured temperature (ambient temperature) of 25 degrees Celsius.

FIG. 2 is a diagram showing a drug sustained-release rate at a measured temperature (ambient temperature) of 37 degrees Celsius.

Mode for Carrying Out the Invention

Hereinafter, the present invention will be described in detail.

A drug sustained-release medical device of the present invention is one including an amphiphilic hydrogel retaining a medicinal substance. In the drug sustained-release medical device of the present invention, a sustained release rate of the medicinal substance is less than or equal to 50 wt% 24 hours after the release of the medicinal substance begins. It is preferred that the sustained release rate of the medicinal substance be less than or equal to 25 wt% four hours after the release of the medicinal substance begins, and be less than or equal to 50 wt% 24 hours after the release of the medicinal substance begins.

The sustained release rate of the medicinal substance is represented in percentage by mass (wt%), and is calculated by the following relational formula:

$$\text{Sustained Release Rate of Medicinal Substance (wt\%)} = \text{(Amount of Medicinal Substance Released After Predetermined Period of Time Has Passed / Total Amount of Medicinal Substance Captured)} \times 100$$

The total amount of the medicinal substance captured is calculated as a value obtained by immersing the drug sustained-release medical device of the present invention in a solvent in which the medicinal substance can easily dissolve; removing the device 24 hours after the device is kept therein; and determining the amount of the medicinal substance in the immersion fluid with the use of high performance liquid chromatographs (HPLC) and methods known to a person of ordinary skill in the art, such as calibration curve method and internal standard method.

The amount of the medicinal substance released after a predetermined period of time has passed is calculated as a value obtained by immersing the drug sustained-release medical device of the present invention in saline; sampling the immersion fluid after a predetermined period of time has passed; and determining the amount of the medicinal substance in the immersion fluid according to a normal method known to a person of ordinary skill in the art with the use of HPLC.

The total amount of the medicinal substance captured, as well as the amount of the medicinal substance released after a predetermined period of time has passed, can be calculated by means of a detailed method according to Examples as mentioned later on.

The amphiphilic hydrogel that constitutes the drug sustained-release medical device of the present invention is not specifically restricted as long as the sustained release rate of the medicinal substance is less than or equal to 50 wt% 24 hours after the release of the medicinal substance begins. For example, the amphiphilic hydrogel may include a copolymer of silicone-containing monomer and ionic monomer. In this case, in the amphiphilic hydrogel, the silicone-containing monomer functions as a hydrophobic unit, while the ionic monomer functions as a hydrophilic unit.

The ionic monomer that functions as a hydrophilic unit is not specifically restricted as long as the ionic monomer includes an ionic functional group, and is compatible with the silicone-containing monomer. For example, the ionic monomer may include a monomer that contains an ionic group in a side chain. According to the present invention, anionic monomers are preferably employed; it is more preferred that, among the anionic monomers, a monomer containing a carboxyl group be employed. In a concrete example, the ionic monomers include: (meth)acrylic acid; 2- (meth) acryloyloxyethyl succinic acid; 2-(meth)acryloyloxyethyl hexahydrophthalic acid; 2-(meth)acryloyloxyethyl phthalic acid; and 2-(meth)acryloyloxyethyl-2-hydroxyethyl-phthalic acid. The above substances may be employed independently, or two or more of the above substances may be employed in combination.

The ionic monomer, which is a hydrophilic unit, is able to form an ionic bond with a water-soluble medicinal substance containing to-be-paired ionic groups, and keep the medicinal substance in the amphiphilic hydrogel. The amount of the ionic monomer blended is preferably in the range of 1 wt% to 20 wt%. If the amount is less than 1 wt%, the amount of the water-soluble medicinal substance kept in the hydrogel is so small that effective treatment cannot be carried out. If the amount exceeds 20 wt%, the shape stability and mechanical strength of the hydrogel decrease. In view of the above fact, it is more preferred that the amount of the ionic monomer blended be in the range of 5 wt% to 15 wt%.

The silicone-containing monomer that functions as a hydrophobic unit is a monomer whose molecular structure includes a silicone compound as a copolymerization component. In the present invention, the monomer is preferably a silicone-containing monomer whose molecular structure includes an alkylsiloxy group.

The drug sustained-release medical device of the present invention includes the silicone-containing monomer as a hydrophobic unit, which helps improve oxygen permeability of the device, as well as reduce the exchange efficiency between the water portion in the amphiphilic hydrogel and lacrimal fluid with the help of repulsion action against the hydrophobic unit. As a result, the amount of the medicinal substance flowing out into the lacrimal fluid is inhibited. In particular, the hydrophobicity of the silicone-containing monomer can realize a high level of inhibitory effect. Furthermore, the silicone-containing monomer whose molecular structure includes an alkylsiloxy group can cause a high steric barrier, making it possible to inhibit the exchange efficiency in a more effective manner.

In order to enhance the effect of inhibiting the exchange efficiency between the water portion inside the amphiphilic hydrogel and lacrimal fluid, the number of alkyl groups in the alkylsiloxy group that the silicone-containing monomer contains is preferably more than or equal to "mono," more preferably more than or equal to "di," and still more preferably more than or equal to "tri." For the same reason, the number of alkylsiloxy groups is one, two, or three.

In a concrete example, the silicone-containing monomers for example include:

α-mono(methacryloyloxymethyl)polydimethylsiloxane;
α,ω-di(methacryloxymethyl)polydimethylsiloxane;
α-mono(3-methacryloyloxypropyl)polydimethylsiloxane;

α,ω-di(3-methacryloyloxypropyl)polydimethylsiloxane;
α-mono(3-methacryloyloxybutyl)polydimethylsiloxane;
α,ω-di(3-methacryloyloxybutyl)polydimethylsiloxane;
α-monovinylpolydimethylsiloxane;
α,ω-divinylpolydimethylsiloxane;
3-tris(trimethylsiloxy)silylmethyl(meth)acrylate;
3-tris(trimethylsiloxy)silylpropyl(meth)acrylate;
3-methylbis(trimethylsiloxy)silylmethyl(meth)acrylate;
3-methylbis(trimethylsiloxy)silylpropyl(meth)acrylate;
3-trimethylsiloxydimethylsilylmethyl(meth)acrylate;
3-trimethylsiloxydimethylsilylpropyl(meth)acrylate; and 3-methyldimethoxysilylpropyl(meth)acrylate. The above substances may be employed independently, or two or more of the above substances may be employed in combination.

The amount of the silicone-containing monomer blended is preferably in the range of 1 wt% to 25 wt%. If the amount is less than 1 wt%, the hydrophobicity is so weak that the exchange efficiency cannot be suppressed. If the amount exceeds 25 wt%, the compatibility with a hydrophilic monomer decreases, making it impossible to obtain a uniform solution at the time when a monomer is prepared. In view of the above fact, it is more preferred that the amount of the silicone-containing monomer blended be in the range of 5 wt% to 15 wt%.

In a process of constituting the drug sustained-release medical device of the present invention, in addition to the above components, a cross-linking monomer may be employed. In a concrete example, the cross-linking monomers include: ethyleneglycoldi(meth)acrylate; methylenebis(meth)acrylamide; 2-hydroxy-1,3-di(meth)acryloxypropane; and trimethylolpropanetri(meth)acrylate. The amount of the cross-linking monomer blended is preferably 0.1 wt% to 4.0 wt% relative to the total amount of monomers used. The blending of the cross-linking monomer enables formation of a network structure of the hydrogel, and adjustment of mechanical strength.

In the process of constituting the drug sustained-release medical device of the present invention, with the aim of adjusting physical properties of the device such as the water content ratio, diffusion coefficients, and the amount of medicinal substances retained, additionally optional copolymerizable monomer may be employed. The optional copolymerizable monomers can be blended without being specifically restricted as long as the monomers have compatibility with an ionic monomer and a silicone monomer. For example, the copolymerizable monomers include: methyl(meth)acrylate; 2-hydroxyethyl (meth) acrylate; 2-hydroxypropyl(meth)acrylate; isobutyl(meth)acrylate; 2, 2, 2-trifluoroethyl(meth)acrylate; and cyclohexyl(meth)acrylate.

The drug sustained-release medical device of the present invention can be produced by a combination of steps known to a person of ordinary skill in the art. Although not specifically restricted, for example, the following steps may be included:

a step of adding a polymerization initiator to a mixture of monomers that constitute an amphiphilic hydrogel, and obtaining a liquid monomer mixture by stirring and dissolving; a step of placing the obtained liquid monomer mixture into a desired forming die to obtain a copolymer through copolymerization reaction; a step of cooling the copolymer, separating the copolymer from the forming die, and cutting and polishing the copolymer if needed followed by obtaining a hydrogel through hydration swelling of the formed copolymer; and
a step of obtaining a drug sustained-release medical device, which retains a medicinal substance inside the hydrogel, by immersing the obtained hydrogel in a solution in which a medicinal substance is dissolved.

As for the polymerization initiator, those known to a person of ordinary skill in the art can be widely employed. For example, the following substances may be employed: peroxide-based polymerization initiators, such as lauroyl peroxide, cumene hydroperoxide, and benzoyl peroxide, which are a typical radical polymerization initiator; and azo-based polymerization initiators, such as azobisdimethylvaleronitrile and azobisisobutyronitrile. The amount of the polymerization initiator added is not specifically restricted as long as the amount is sufficient to promote the copolymerization reaction of monomers. For example, the amount of the polymerization initiator added is preferably around 10 ppm to 7,000 ppm relative to the total amount of monomers.

For example, the step of obtaining the copolymer may be carried out by placing the liquid monomer mixture into a forming die of the medical device, which is made from plastics and the like; increasing the temperature in stages, or continuously, within the range of 25 to 120 degrees Celsius in a thermostatic chamber or the like; and completing the polymerization for 5 to 120 hours. As for the copolymerization, ultraviolet rays, electron rays, gamma rays, or the like may also be used.

For example, the step of obtaining the copolymer may apply solution polymerization, which is realized by adding various kinds of solvent to the liquid monomer mixture. The solvents that can be employed for the solution polymerization are not specifically restricted as long as the solvents are those known to a person of ordinary skill in the art as solvents employed in the solution polymerization. For example, the solvents include: water; tetrahydrofuran; glycerin; dimethylformamide; dimethyl sulfoxide; and 1, 4-dioxane. The amount of the solvent used is normally within the range of 1 wt% to 60 wt% relative to the total amount of monomers. However, for the purpose of ensuring that the finally obtained medical device has a better shape, it is preferred that the amount be in the range of 5 wt% to 40 wt%.

For example, the step of obtaining the hydrogel may be carried out by cooling the forming die after the copolymerization reaction down to an ambient temperature; separating the copolymer from the forming die; cutting and polishing the copolymer if needed; and then subjecting the copolymer to the hydration swelling to obtain the hydrogel. As for the liquid (swelling liquid) that is employed for the step, those known to a person of ordinary skill in the art can be widely employed. For example, the liquids include water, saline, and isotonic buffers. The swelling treatment for the hydration swelling can be achieved, for example, by immersing the copolymer in a swelling liquid that is heated to 60 to 100 degrees Celsius for a certain period of time. The swelling treatment enables unreacted monomers contained in the polymer to be removed.

For example, the step of obtaining the drug sustained-release medical device may be carried out by immersing the hydrogel obtained by the swelling treatment in a solution in which a medicinal substance is dissolved. The medicinal substance that is employed in the process of constituting the drug sustained-release medical device of the present invention is kept inside the hydrogel by ionic bond with the ionic group, which is provided by the ionic monomer. Therefore, the medicinal substance employed is determined based on properties of the ionic monomer. However, it is preferred that the medicinal substance employed be a water-soluble medicinal substance. In the present invention, as the ionic monomer, an anionic monomer is preferably employed. Therefore, it is preferred that the water-soluble medicinal substance have a structure containing a cationic functional group.

As for the water-soluble medicinal substance that includes a cationic functional group, the following substance is preferred: a medicinal substance that intramolecularly includes at least one quaternary ammonium base, or primary to tertiary amine base. More specifically, the water-soluble medicinal substances include ofloxacin, levofloxacin, norfloxacin, and gatifloxacin.

The solvents that are used to dissolve the medicinal substance include water, hydrophilic solvents, and mixed solvents of water and hydrophilic solvents. For example, the hydrophilic solvents include alcohols, such as ethanol, methanol, isopropanol, and n-butanol; and dimethyl sulfoxide.

The concentration of the medicinal substance in the medicinal-substance solution, which is obtained by dissolving the medicinal substance in the solvent, is not specifically restricted as long as the amount can be captured by the amphiphilic hydrogel, or is greater than the level at which the amphiphilic hydrogel can capture. For example, the concentration may be appropriately set by a person of ordinary skill in the art based on the solubility of the medicinal substance, the lowest effective concentration, at which the medicinal effects appear, the maximum safe concentration, the medicinal-substance release period, the device administration period, or the like.

The drug sustained-release medical device of the present invention can be formed into various shapes, and applied in various fields. More specifically, the drug sustained-release medical device can be turned into a contact lens as the shape thereof is processed so as to be adjusted to the curvature of the anterior segment of the eye. Furthermore, as a ring-shaped ophthalmic device that is adjusted to the curvature of the sclera portion, the drug sustained-release medical device can be turned into an intraocular lens (IOL) as the shape thereof is so processed as to fit an intraocular shape. If the shape of the drug sustained-release medical device is formed into a sheet, the drug sustained-release medical device can serve as an adhesive skin patch, such as poultice. If the shape of the drug sustained-release medical device is formed into a capsule, the drug sustained-release medical device can serve as an orally-administered agent or the like.

Examples

Hereinafter, the present invention will be described in more detail in Examples. However, the present invention is not limited to Examples.

The medical device's monomer solutions according to Examples and Comparative Examples of the present invention were prepared according to the composition amounts shown in Table 1, and were stirred for about one hour with sufficient nitrogen substitution. After the stirring, each liquid monomer mixture was poured into a forming die for ophthalmic lens. Then, the temperature was raised within the range of 50 to 100 degrees Celsius over 25 hours, and the polymer was obtained. After the obtained polymer was cooled down to an ambient temperature, the polymer was taken out of the die. Then, the polymer was immersed in distilled water at about 80 degrees Celsius for about four hours, thereby subjecting the polymer to hydration swelling. The obtained medical devices all were colorless and transparent hydrogel.

Such medical devices were immersed in 10 ml of a medicinal-substance solution that was prepared in advance according to the composition amounts shown in Table 1, at 25 degrees Celsius for 48 hours so that the cationic medicinal substance was retained. After that, the medical devices were assessed in the following respects.

(Measurement of water content ratio)

After the medical devices were swelled and excess water was wiped off, the weight ($W_1$) of the devices containing water was measured. Then, the medical devices were dried in a drying machine overnight at 60 degrees Celsius. The weight ($W_2$) of the dried devices was measured. Based on each weight of the devices, the water content ratio was calculated.

$$\texttt{Water Content Ratio (Percentage by Weight) = [(W_1-W_2)/W_1]\times100}$$

(Assessment of the amount of the medicinal substance captured)

The medical devices were immersed in 10mL of methanol at 25 degrees Celsius, and were left therein for 24 hours. After the devices were taken out, the amount of the medicinal substance contained in methanol was determined by using HPLC (JASCO Corporation; Column Phenomenex Luna C18 5$\mu$ 150x4.6 mm). The obtained values were used as the amounts of medicinal substances captured.

(Assessment of drug sustained-release performance)

The medical devices were immersed in 10mL of saline at 25 or 37 degrees Celsius. For each medical device, after a predetermined period of time (4, 8, 24, or 72 hours) had passed, the immersion fluid was sampled; the amount of the medicinal substance contained in the immersion fluid was determined by using HPLC (JASCO Corporation; Column Phenomenex Luna C18 5$\mu$ 150$\times$4.6mm). The resultant values were expressed in percentage relative to the total amount of the medicinal substance captured, and the drug sustained-release performance was assessed.

$$\texttt{Sustained-release Rate (\%) = (Amount of Medicinal Substance}$$
$$\texttt{Released in a Sustained Manner After Each Period of Time has Passed}$$
$$\texttt{/ Total Amount of Medicinal Substance Captured) }\times\texttt{100}$$

(Examples 1 to 3 and Comparative Examples 1 and 2)

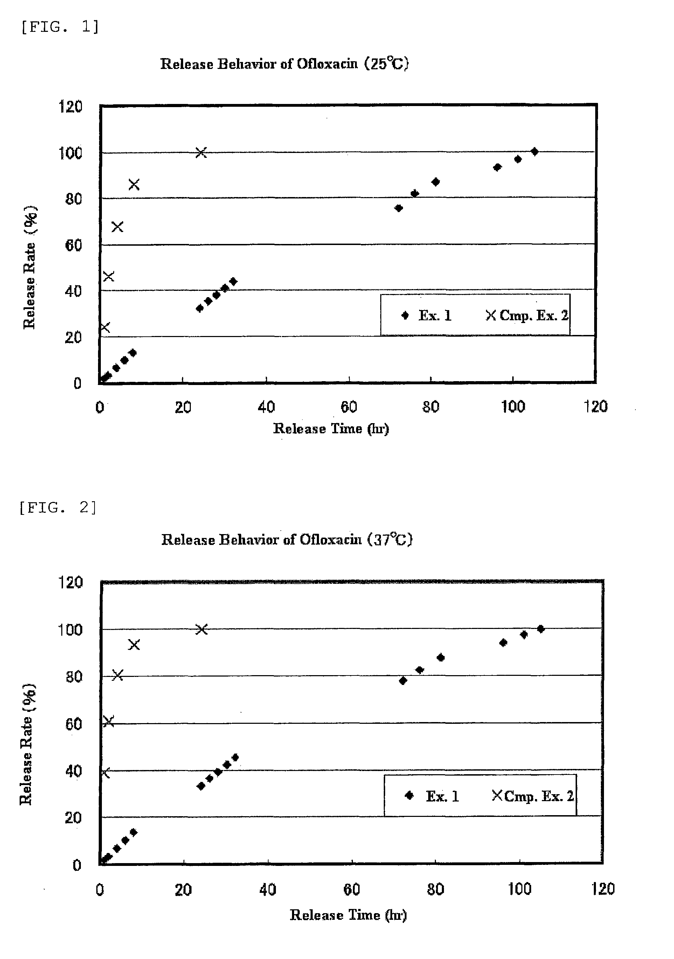

Table 1, and FIGS. 1 and 2 show the results of assessment for the medical devices obtained. In Examples 1 to 3, the initial burst was suppressed. Moreover, the sustained release of the medicinal substance was possible over 72 hours, suggesting the sustained release performance of the medicinal substance be under control.

In Comparative Example 1, a therapeutically sufficient amount of the medicinal substance could not be retained. It can be assumed that such a result was caused by the fact that an ionic monomer was not included as a component of the medical device.

In Comparative Example 2, the release of the medicinal substance could not be controlled. It can be assumed that such a result was caused by the fact that a silicone-containing monomer was not included as a component of the medical device.

As described above, according to the present invention, since the ionic monomer is included as a component of the drug sustained-release medical device, it becomes possible to retain a therapeutically effective dose of medicinal substances. Furthermore, since the silicone-containing monomer is included, it becomes possible to control the amount of retained medicinal substances that are released, as well as to release the medicinal substances in a sustained manner over a long period of time with the initial burst suppressed.

**EP 2 650 021 B1**

[Table 1]

|  |  |  | Ex. 1 | Ex. 2 | Ex. 3 | Cmp. Ex. 1 | Cmp. Ex 2 |
|---|---|---|---|---|---|---|---|
| Hydrogel Composition | Ionic group-containing monomer | MAA | 10 | 10 | 10 | - | 10 |
|  |  | HOMS | - | 5 | - | - | - |
|  | Silyl group-containing monomer | TM0701 | 10 | 10 | - | 10 | - |
|  |  | FM0711 | - | 5 | - | - | - |
|  |  | STA0198 | - | - | 10 | - | - |
|  | Copolymerizable monomer | 8F | 30 | - | 25 | 20 | - |
|  |  | DMAA | 20 | - | - | - | - |
|  |  | NVP | - | 10 | 30 | 44.5 | - |
|  |  | HEMA | 34.5- | 44 | 24.5 | 25 | 75 |
|  |  | DNBAAm | - | 15 | - | - | - |
|  | Crosslinking agent | EDMA | 0.5 | - | 0.5 | 0.5 | 3 |
|  |  | 1, 9-NDA | - | 1 | - | - | 12 |
|  | Initiator (External) | AIBN | 0.3 | 0.3 | 0.3 | 0.3 | 0.3 |
| Medicinal-substance solution composition | ofloxacin | | 0.3 | - | 0.3 | 0.3 | 0.3 |
|  | gatifloxacin | | - | 0.3 | - | - | - |
|  | sodium chloride | | 0.85 | 0.85 | 0.85 | 0.85 | 0.85 |
|  | hydrochloric acid | | proper quantity | proper quantity | proper quantity | proper quantity | proper quantity |
|  | purified water | | 100mL | 100mL | 100mL | 100mL | 100mL |
| Assessment results | Water content ratio | | 36 | 34 | 38 | 32 | 38 |
|  | Amount of medicinal substance captured ($\mu$g/g) | | 43572 | 47032 | 23788 | 9018 | 39700 |
|  | Sustained-release rate (25°C) | After 4 hours | 7% | - | - | 27% | 68% |
|  |  | After 8 hours | 13% | - | - | 38% | 86% |
|  |  | After 24 hours | 32% | - | - | 69% | 100% |
|  |  | After 72 hours | 76% | - | - | 86% | - |
|  | Sustained-release rate (37°C) | After 4 hours | 7% | 22% | 24% | 28% | 81% |
|  |  | After 8 hours | 14% | 29% | 35% | 40% | 95% |
|  |  | After 24 hours | 37% | 42% | 53% | 70% | 100% |
|  |  | After 72 hours | 78% | 68% | 88% | 91% | - |

The meaning of the abbreviations in Examples and Comparative Examples is as follows:

9

MAA; methacrylic acid

MOESA; 2- methacryloyloxyethyl succinic acid

TM0701; Silaplane TM-0701 (manufactured by Chisso Corporation; 3-tris (trimethylsiloxy) silylpropylmethacrylate)

FM0711; Silaplane FM-0711 (manufactured by Chisso Corporation: terminal methacryloyl oxypolydimethylsiloxane)

SIA0198: 3-methyldimethoxy silylpropylacrylate (manufactured by Gelest, Inc)

8F: 1H, 1H, 5H-octafluoropentyl acrylate

DMAA; dimethylacrylamide

NVP; N-vinylpyrrolidone

HEMA; 2-hydroxyethyl methacrylate

DNBAAm; N,N-di-n-butyl methacrylamide

EDMA; ethylene glycol dimethacrylate

1,9-NDA; 1,9-nonane diol dimethacrylate

AIBN; azobisisobutyronitrile

## Claims

1. A drug sustained-release medical contact lens comprising an amphiphilic hydrogel for retaining a water-soluble medicinal substance comprising a cationic functional group, wherein a sustained-release rate of the medicinal substance is less than or equal to 50 wt% 24 hours after a release of the medicinal substance begins, and the amphiphilic hydrogel is a copolymer obtained through copolymerization reaction of liquid monomer mixture comprising 5wt% to 15wt% of silicone-containing monomer and 5wt% to 15wt% of anionic monomer comprising a carboxyl group.

2. The drug sustained-release medical contact lens according to claim 1, wherein the sustained-release rate of the medicinal substance is less than or equal to 25 wt% four hours after the release of the medicinal substance begins.

3. The drug sustained-release medical contact lens according to claim 1, wherein the liquid monomer mixture further comprises a cross-linking monomer the amount of which is 0.1 wt% to 4.0 wt% relative to the total amount of monomers used.

4. The drug sustained-release medical contact lens according to claim 1, wherein the amphiphilic hydrogel comprises a silicone-containing monomer comprising 1 to 3 monoalkylsiloxy groups, dialkylsiloxy groups, or trialkylsiloxy groups.

## Patentansprüche

1. Medizinische Kontaktlinse, die verzögert Wirkstoff freisetzt, umfassend ein amphiphiles Hydrogel zum Zurückhalten einer wasserlöslichen medizinischen Substanz, umfassend eine kationische, funktionelle Gruppe, wobei der Prozentsatz der verlängerten Freisetzung der medizinischen Substanz 24 Stunden nach einem Beginn des Freisetzens der medizinischen Substanz weniger oder gleich 50 Gew.-% beträgt, und wobei das amphiphile Hydrogel ein Copolymer ist, das durch eine Copolymerisationsreaktion einer flüssigen Monomermischung erhalten wird, die 5 Gew.-% bis 15 Gew.-% eines silikonhaltigen Monomers und 5 Gew.-% bis 15 Gew.-% eines anionischen, eine Carboxylgruppe enthaltenden Monomers umfasst.

2. Medizinische Kontaktlinse, die verzögert Wirkstoff freisetzt, gemäß Anspruch 1, wobei der Prozentsatz der verlängerten Freisetzung der medizinischen Substanz vier Stunden nach dem Beginn des Freisetzens der medizinischen Substanz weniger oder gleich 25 Gew.-% beträgt.

3. Medizinische Kontaktlinse, die verzögert Wirkstoff freisetzt, gemäß Anspruch 1, wobei die flüssige Monomermischung des Weiteren ein vernetzendes Monomer umfasst, dessen Menge 0,1 Gew.-% bis 4,0 Gew.-% in Bezug auf die Gesamtmenge der verwendeten Monomere beträgt.

4. Medizinische Kontaktlinse, die verzögert Wirkstoff freisetzt, gemäß Anspruch 1, wobei das amphiphile Hydrogel ein silikonhaltiges Monomer umfasst, das 1 bis 3 Monoalkylsiloxygruppen, Dialkylsiloxygruppen oder Trialkylsiloxygruppen umfasst.

**Revendications**

1. Lentille de contact médicale à libération prolongée de médicament comprenant un hydrogel amphiphile pour retenir une substance médicamenteuse hydrosoluble comprenant un groupe fonctionnel, dans laquelle une vitesse de libération prolongée de la substance médicamenteuse est inférieure ou égale à 50 % en poids par 24 heures après qu'une libération de la substance médicamenteuse a commencé, et l'hydrogel amphiphile est un copolymère obtenu par une réaction de copolymérisation d'un mélange de monomères liquide comprenant de 5 à 15 % en poids d'un monomère contenant une silicone et de 5 à 15 % en poids d'un monomère anionique comprenant un groupe carboxy.

2. Lentille de contact médicale à libération prolongée de médicament selon la revendication 1, dans laquelle la vitesse de libération prolongée de la substance médicamenteuse est inférieure ou égale à 25 % en poids par 4 heures après que la libération de la substance médicamenteuse a commencé.

3. Lentille de contact médicale à libération prolongée de médicament selon la revendication 1, dans laquelle le mélange de monomères liquide comprend en outre un monomère de réticulation dont la quantité est de 0,1 à 4,0 % en poids par rapport à la quantité totale des monomères utilisés.

4. Lentille de contact médicale à libération prolongée de médicament selon la revendication 1, dans laquelle l'hydrogel amphiphile comprend un monomère contenant une silicone comprenant de 1 à 3 groupes monoalkylsiloxy, dialkylsiloxy, ou trialkylsiloxy.

[FIG. 1]

Release Behavior of Ofloxacin (25°C)

[FIG. 2]

Release Behavior of Ofloxacin (37°C)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011061735 A
- JP 2009204770 A
- JP 2004305313 A
- EP 1128191 A2
- EP 0351364 A2